# EUROPEAN PATENT SPECIFICATION

(11) **EP 2 462 094 B1**
(45) Date of publication and mention of the grant of the patent: **21.12.2016**
(21) Application number: 10751827.6
(22) Date of filing: 06.08.2010
(51) Int. Cl.: B01J 31/02, B01J 31/26, B01J 31/30, C07C 2/58

(54) **PROCESS FOR PREPARING AN ALKYLATE**
PROZESS ZUR HERSTELLUNG EINES ALKYLATES
PROCÉDÉ POUR PRÉPARER UN ALKYLATE

(30) Priority: 06.08.2009 WO PCT/CN2009/000891
(43) Date of publication of application: 13.06.2012
(73) Proprietor: Shell Internationale Research Maatschappij B.V., 2596 HR The Hague (NL)
(72) Inventor: LIU, Zhichang, Beijing 102249 (CN); XU, Chunming, Beijing 102249 (CN); ZHANG, Rui, Beijing 102249 (CN); MENG, Xianghai, Beijing 102249 (CN); PATRONI, Ana, Cecilia, 1031 HW Amsterdam (NL); KLUSENER, Peter, Anton, August, 1031 HW Amsterdam (NL); VAN DEN BOSCH, Albertus, Vincentius, Petrus, 1031 HW Amsterdam (NL)
(74) Representative: Matthezing, Robert Maarten
(86) International application number: PCT/EP2010/061509
(87) International publication number: WO 2011/015661

(56) References cited:
- US-A1- 2004 133 056

## Description

### Field of the invention

The present invention provides a process preparing an alkylate.

### Background of the invention

There is an increasing demand for alkylate fuel blending feedstock. As a fuel-blending component alkylate combines a low vapour pressure with high octane properties.

Almost all alkylate is produced by reacting isobutane with butene in the presence of a suitable acidic catalyst. The most used catalysts are HF and sulphuric acid, although other catalysts such a solid acid catalyst have been reported. Recently, the alkylation of isoparaffins with olefins using an acidic ionic liquid catalyst has attracted attention as an alternative to HF and sulphuric acid catalysed alkylation processes.

In for instance US7285698 a process for manufacturing an alkylate oil is disclosed, which uses a composite ionic liquid catalyst to react isobutane with a butene. In the process of US7285698, isobutane and butene are supplied to a reactor and the alkylate is formed by contacting the reactants with a composite ionic liquid under alkylation conditions. The reactor effluent is separated and the ionic liquid phase is recycled to the reactor while the hydrocarbon phase is treated to retrieve the alkylate.

In for instance Liu et al. (Z.Liu, R.Zhang, C.Xu, R.Xia, Ionic liquid alkylation process produces high-quality gasoline, Oil and Gas Journal, vol 104, Issue 40, 2006) it is mentioned that it is possible to retrofit a sulphuric acid alkylation unit for use of an IL catalyst. However, it is mentioned that it is necessary to modify the settler unit to improve the separation of the reactor effluent, i.e. the ionic liquid catalyst and hydrocarbon phase.

In US2005/0119423, a process for preparing polyalphaolefins is disclosed using a ionic liquid catalyst. In the process of US2005/0119423, the reactor effluent is provided to a separator to obtain a polyalphaolefin-containing phase, which still comprises remaining ionic liquid catalyst. It is suggested in US2005/0119423, to mix the polyalphaolefin-containing phase with water to deactivate the catalyst and subsequently separate the polyalphaolefin-containing phase from the water in two additional sequential separation steps.

US 2004/0133056 discloses a process for producing an alkylate oil using an ionic liquid as catalyst.

There is a need in the art for an ionic liquid alkylation process, which provides an improved separation of the reactor effluent.

### Summary of the invention

It has now been found that the reactor effluent of an ionic liquid alkylation reactor can be efficiently separated in an acidic ionic liquid phase and an hydrocarbon phase using a staged separation.

Accordingly, the present invention provides a process for preparing an alkylate comprising contacting in a reactor a hydrocarbon mixture comprising at least an isoparaffin and an olefin with an acidic ionic liquid catalyst under alkylation conditions to obtain an alkylate, which process further comprises:
- withdrawing an alkylate-comprising reactor effluent from the reactor, wherein the reactor effluent comprises an ionic liquid phase and a hydrocarbon phase;
- separating at least part the reactor effluent into an ionic liquid phase effluent and a multiple-phase effluent in a first separation unit;
- separating at least part of the multiple-phase effluent into a hydrocarbon phase effluent and another effluent in a second separation unit; and
- recycling at least part of the ionic liquid phase effluent to the reactor; in which process the ionic liquid phase effluent comprises solids, which process further comprises:
- providing the ionic liquid phase effluent to a separation unit suitable for separating solids and liquids; and
- removing at least part of the solids from the ionic liquid phase effluent.

An advantage of the present invention is that there is no need to add water or additional compounds to the hydrocarbon phase comprising the alkylate. In addition, no catalyst is purposely deactivated or otherwise destroyed resulting in a lower catalyst consumption of the process.

### Brief description of the drawings.

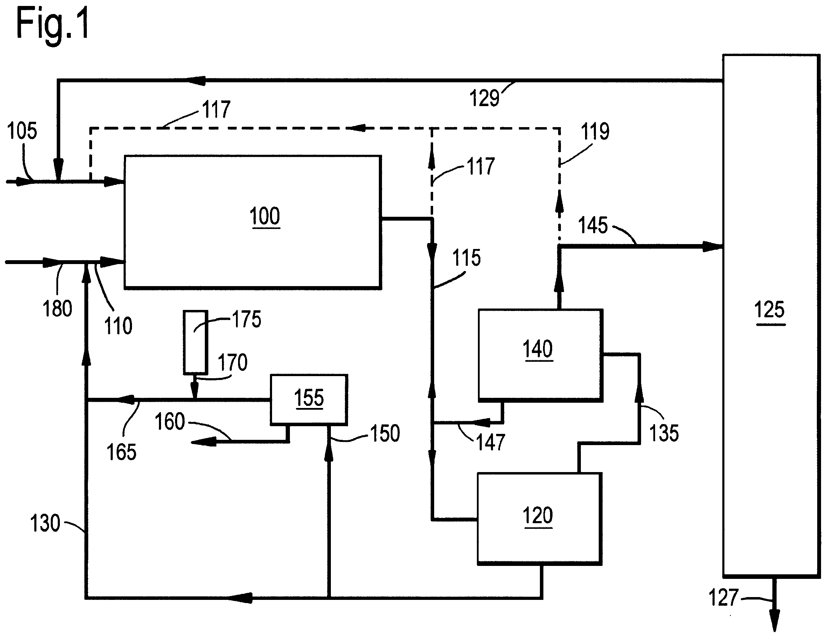

In Figure 1 a schematic representation is given of a process according to the invention.

### Detailed description of the invention

In the process according to the invention an alkylate is prepared by reacting an isoparaffin with an olefin. The obtained alkylate is particularly suitable for gasoline blending purposes. In the process according to the invention the isoparaffin and the olefin are provided to a reactor. In the reactor a hydrocarbon mixture comprising isoparaffin and olefin is contacted with a catalyst suitable for alkylation. The hydrocarbon mixture comprises olefin typically supplied externally, i.e. fresh olefin, and comprises isoparaffin. The isoparaffin may be externally supplied isoparaffin, i.e. fresh isoparaffin, and/or isoparaffin, which is recycled from any other part of the process. The fresh isoparaffin and olefin may be supplied to the process separately, however typically the fresh isoparaffin and the fresh olefin are provided to the reactor as a mixture comprising isoparaffin and olefin. In the present invention the catalyst is an acidic ionic liquid catalyst (herein below also referred to as catalyst).

Ionic liquids are known in the art for their ability to catalyse alkylation reactions. The catalyst used in the present invention is a composite ionic liquid comprising cations derived from a hydrohalide of an alkyl-containing amine or pyridine. Preferably, the cations comprise nitrogen atoms, which are saturated with four substituents, among which there is at least one hydrogen atom and one alkyl group. More preferably, the alkyl substituent is at least one selected from methyl, ethyl, propyl, butyl, amyl, and hexyl groups. Examples of suitable cations include triethyl-ammonium (NEt₃H⁺) and methyl- diethyl-ammonium cations (MeNEt₂H⁺) or

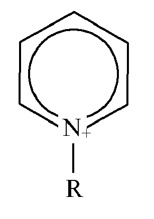

The anions of the composite ionic liquid are preferably aluminium based Lewis acids, in particular aluminium halides, preferably aluminium (III) chloride. Due the high acidity of the aluminium chloride Lewis acid it is preferred to combine the aluminium chloride, or other aluminium halide, with a second or more metal halide, sulphate or nitrate to form a coordinate anion, in particular a coordinate anion derived from two or more metal halides, wherein at least one metal halide is an aluminium halide. Suitable further metal halides, sulphates or nitrates, may be selected from halides, sulphates or nitrates of metals selected from the group consisting of Group IB elements of the Periodic Table, Group IIB elements of the Periodic Table and transition elements of the Periodic Table. Examples or suitable metals include copper, iron, zinc, nickel, cobalt, molybdenum, or platinum. Preferably, the metal halides, sulphates or nitrates, are metal halides, more preferably chlorides or bromides, such as copper (I) chloride, copper (II) chloride, nickel (II) chloride, iron (II) chloride. Preferably, the molar ratio of the aluminium compound to the other metal compounds in the range of from 1:100-100:1, more preferably of from 1:1-100:1, or even more preferably of from 2:1-30:1. By using a coordinate anion comprising aluminium and another metal improved alkylate product may be obtained. A method for preparing such catalyst is for instance described in US7285698. A particularly preferred catalyst is an acidic ionic liquid catalyst comprising a cation derived from triethyl-ammonium chloride and a coordinate anion derived from aluminium(III) chloride and copper(II) chloride.

As mentioned hereinabove, the hydrocarbon mixture comprising isoparaffin and olefin is contacted with the catalyst. The hydrocarbon mixture is mixed in the reactor with the catalyst to form a reaction mixture. As the reaction progresses the reaction mixture will, besides hydrocarbon reactants and acid ionic liquid, additionally comprise products. Mixing of the hydrocarbon mixture and the catalyst may be done by any suitable means for mixing two or more liquids, including dynamic and static mixers. In contact with the catalyst, the isoparaffins and olefins react under alkylation condition to form an alkylate.

An alkylate-comprising reactor effluent (further also referred to a reactor effluent) is withdrawn from the reactor. The reactor effluent comprises a hydrocarbon phase and an ionic liquid phase. Reference herein to a hydrocarbon phase is to a phase comprising hydrocarbons and in the range of from 0 to 5 vol%, preferably, of from 0 to 3 vol% of dissolved acidic ionic liquid, more preferably of from 0 to 0.5 vol% of dissolved acidic ionic liquid based on the total liquid weight of the hydrocarbon phase. Preferably, the hydrocarbon phase comprises in the range of from 95 to 100 vol% of hydrocarbons, preferably of from 97 to 100 vol% of hydrocarbon, based on the total liquid weight of the hydrocarbon phase. Reference herein to an ionic liquid phase is to an ionic liquid phase comprising acidic ionic liquid and in the range of from 0 to 20 vol%, preferably 0 to 10 vol% of dissolved hydrocarbons based on the total liquid weight to the ionic liquid phase. Preferably, the ionic liquid phase comprises in the range of from 80 to 100 vol%, preferably 90 to 100 vol% of acidic ionic liquid based on the total liquid weight to the ionic liquid phase. It will be appreciated that, although the hydrocarbon phase and the ionic liquid phase are immiscible, it is however possible that small amounts of hydrocarbons dissolve in the ionic liquid phase and vice versa that small amounts of acidic ionic liquid catalyst dissolve in the hydrocarbon phase.

The reactor effluent still comprises a substantial amount of unreacted isoparaffin. Therefore, part of the reactor effluent may be recycled to the reactor to maintain a high molar ratio of isoparaffin to olefin. In the process according to the invention at least part of the reactor effluent is provided to a first separation unit to separate the reactor effluent.

It will be appreciated that typical industrial scale separation units cannot separate the reactor effluent into two or more effluents comprising only one phase, e.g. a hydrocarbon phase or ionic liquid phase. However, it has now been found that it is possible to separate the reactor effluent into an ionic liquid phase effluent and a multiple-phase effluent comprising hydrocarbons and acidic ionic liquid. Reference herein to an ionic liquid phase effluent is to an effluent comprising ionic liquid phase and in the range of from 0 to 20 vol%, preferably 0 to 10 vol% of a hydrocarbon phase based on the total liquid weight to the ionic liquid phase effluent. Preferably, the ionic liquid phase effluent comprises in the range of from 80 to 100 vol%, preferably 90 to 100 vol% of ionic liquid phase based on the total liquid weight to the ionic liquid phase effluent.

In the above-described separation of two immiscible phases, an essentially pure effluent is withdrawn from the separator comprising predominantly one phase, while it is accepted that the multiple-phase effluent contains significant amounts of both phases. Such a separation is within the normal skills of the skilled person in the art of separations.

The ionic liquid phase effluent is at least in part recycled to the reactor.

In the process according to the invention, at least part of the multiple-phase effluent obtained from the first separation unit is subsequently, without any intermediate chemical treatment, sent to a second separation unit. Reference herein to a chemical treatment is to a treatment resulting in the decomposition of one or more of the compounds in the multiple-phase effluent.

The multiple-phase effluent comprises most if not essentially all of the hydrocarbons, which were part of the reactor effluent. However, as describe hereinabove, the multiple-phase effluent also comprises significant amounts of acidic ionic liquid.

In the second separation unit, the multiple-phase effluent is separated into a hydrocarbon phase effluent and another effluent. Reference herein to a hydrocarbon phase effluent is to an effluent comprising a hydrocarbon phase and in the range of from 0 to 5 vol%, preferably, of from 0 to 3 vol% of an ionic liquid phase, more preferably of from 0 to 0.5 vol% of an ionic liquid phase based on the total weight of the hydrocarbon phase effluent. Preferably, the hydrocarbon phase effluent comprises in the range of from 95 to 100 vol% of hydrocarbon phase, preferably of from 97 to 100 vol% of hydrocarbon phase, based on the total weight of the hydrocarbon phase effluent.

The first and/or second separation unit may by any separation unit suitable to separate two immiscible liquid phases. Examples of suitable separation unit include settler separation units and centrifugal separation units. Preferably, the first and/or second separation units are centrifugal separation units, more preferably cyclone type separation units, as such separation units allow for a fast and continues separation of two immiscible liquid phases. In addition, due to the ability to reduce the time required for the separation compared to conventional settler units, centrifugal separation units can typically be less voluminous, requiring a smaller footprint and, more importantly, a much smaller inventories of liquefied light hydrocarbons, in particular isobutane. Preferably, the cyclone is a hydro-cyclones. Reference herein to a hydro-cyclone is to a cyclone designed for the separation of water-hydrocarbon mixtures. Preferably both the first and the second are cyclone type separation units.

Reference herein to a separation unit is a separation unit comprising one separator, e.g. a cyclone or two or more separators aligned in parallel.

In case the volume of the reactor effluent is high, it is possible to provide two or more combinations of the first and second separation units in parallel.

The hydrocarbon phase effluent still comprises a substantial amount of unreacted isoparaffin. Therefore, part of the hydrocarbon phase effluent may be recycled to the reactor to maintain a high molar ratio of isoparaffin to olefin. At least part of the hydrocarbon phase effluent may be fractionated into at least a hydrocarbon stream comprising alkylate and a stream comprising isoparaffin.

If desired the ionic liquid fraction in the hydrocarbon effluent may be lowered even further by subjecting the hydrocarbon phase effluent to a further physical separation treatment in a further centrifugal separation unit, such as a cyclone, or in a settler unit. Although, a settler unit may be relatively large in volume compared to e.g. a cyclone, if a settler unit is used as a second separator unit, the invention still provides an advantage as the second settler unit will be much smaller than a settler unit in a conventional process, which is used to separate the reactor effluent. Preferably, the process further comprises providing, prior to fractionating the hydrocarbon phase effluent, the hydrocarbon phase effluent to a further settler unit to remove additional ionic liquid.

The stream comprising isoparaffin is generally reused to form part of the isoparaffin feed provided to the process. This may be done by recycling at least part of the stream comprising isoparaffin and combining it with the isoparaffin feed to the process.

The obtained hydrocarbon stream comprising alkylate may be used to prepare avgas or as a blending component for gasoline. Other hydrocarbon streams may also be obtained by fractionation of the essentially pure hydrocarbon effluent, such a n-paraffin-comprising stream.

The hydrocarbon phase effluent may be fractionated using any suitable method to fractionate a hydrocarbon stream. Preferably, the hydrocarbon phase effluent is fractionated using one or more distillation units.

The another effluent obtained from the second separation unit may be recycled to the first separation unit or be combined with the reactor effluent, which is provided to the first separation unit.

It has been observed that during the alkylation reaction solids are formed. Reference, herein to solids is to non-dissolved solid particles. The solids may have any size, however it was found that the solids have an average size of in the range of from 0.1 to 5µm. In particular, at least 50% of the solids have a particle size below 5µm, more particular 80% of the solids have a particle size below 5µm based on the total number of solid particles.

Although, during mixing these solids are dispersed throughout the reaction mixture, upon separation of the reactor effluent it has been found that the solids, i.e. to a large extent, accumulate in the ionic liquid phase effluent. This is due to the high density of the solids. When the ionic liquid phase effluent is, at least in part, recycled to the reactor, the solids accumulate in the reaction mixture, resulting in undesirably high solids content in the reaction mixture. A high solids content in the reaction mixture may for instance result in blockage of pathways or valves in the reactor and pipes to and from the separation unit, due to precipitation of solids. In addition, at high solids content the solids may agglomerate to from large aggregates, resulting in increased blockage risk.

Therefore, it is preferred that in the process according to the invention solids are removed from the ionic liquid phase effluent by providing the ionic liquid phase effluent to a third separation unit suitable for separation of solids and liquids and removing the solids from the essentially pure acidic ionic liquid effluent. In the present invention, at least part of the solids are removed from the ionic liquid phase effluent. Preferably, solids are removed from the ionic liquid phase effluent to an extent that the reaction mixture comprises at most 5wt% of solids, preferably at most 2wt% of solids, based on the total weight of the acidic ionic liquid catalyst in the reactor. By removing the solids from the ionic liquid phase effluent rather than from the reactor effluent, the liquid volume passing through the third separation unit is reduced, thus allowing for the use of a smaller separation unit.

The third separation unit may be any separation unit suitable for removing solids and liquids, including but not limited to filtration, precipitation and centrifugation processes. Such processes are well known in the art.

Due to the specific nature of ionic liquids it is preferred that the removal of the solids is performed at such a temperature that the acidic ionic liquid catalyst is liquid. In particular, it is preferred to remove the solids at a temperature in the range of from 5 to 80°C, more preferably of from 20 to 60°C. By removing the solids at elevated temperatures, the viscosity of the ionic liquid is low, which may be beneficial in view of the decreased time and power input required to obtained separation of the solids from the liquid.

The solids may be removed form the process in any form, typically the solids will be removed in the form of a slurry of solids. Such a slurry may comprise next to the solids for instance some residual acidic ionic liquid.

Optionally, the catalyst in the ionic liquid phase effluent can be contacted with an acid, preferably a hydrogen halide, more preferably hydrogen chloride, to rejuvenate the catalyst. Preferably, the acidic ionic liquid catalyst is rejuvenated by recycling at least part of the ionic liquid phase effluent to the reactor and the at least part of the acidic ionic liquid catalyst in the recycled effluent is rejuvenated by addition of hydrogen chloride to at least part of the recycled effluent.

By contacting the catalyst with hydrogen chloride after removal of most of the hydrocarbons, undesired chlorination of hydrocarbons is reduced. The hydrogen chloride reacts with the acidic ionic liquid catalyst. Hydrogen chloride is added until no hydrogen chloride is consumed any longer, i.e. until saturation. Hydrogen chloride consumption can be followed by monitoring the pressure. Preferably, the addition of hydrogen chloride is done in regular steps, optionally to batches of the ionic liquid to be rejuvenated, while measuring the pressure in between each addition step. By adding the hydrogen chloride in small steps the creation of an undesired hydrogen chloride gas cap upon saturation is reduced.

The addition of hydrogen chloride may be done by injecting the hydrogen chloride into one or more units or into one or more streams passing from one unit to the next. Hydrogen chloride addition may for instance be done using a venturi absorber, preferably a venturi absorber located downstream from the means for removing solids.

As mentioned herein above, although some gaseous hydrogen chloride in the reactor may be tolerated, it is undesired to accumulate unreacted gaseous hydrogen chloride in the reaction system as a result of over-saturation of the acidic ionic liquid with hydrogen chloride. Residual gaseous hydrogen chloride may be purged from the reaction system by for instance flushing with an inert gas such as nitrogen. However, such process would require additional means for providing nitrogen gas and subsequent storage and treatment of hydrogen chloride-contaminated nitrogen gas. In addition, part of the hydrogen chloride is provided for rejuvenation is lost. Preferably, such hydrogen chloride accumulation is reduced by mixing additional spent acidic ionic liquid catalyst, e.g. in the form of a spent catalyst-comprising stream, into the rejuvenated acidic ionic liquid catalyst comprising recycled effluent, i.e. the recycled effluent comprising added hydrogen chloride. Reference, herein to spent acidic ionic liquid catalyst is to an acidic ionic liquid catalyst, which has been used as a catalyst in a chemical reaction and has not yet been rejuvenated with hydrogen chloride. By allowing the spent acidic ionic liquid to react with the gaseous hydrogen chloride present due to initial over-saturation, the remaining hydrogen chloride may be consumed. The spent ionic liquid catalyst may be introduced from an external source, however it is also possible to allow part of the ionic liquid phase effluent to bypass the rejuvenation and subsequently mix the rejuvenated and bypassed streams. For instance it is possible to rejuvenate the ionic liquid phase effluent and subsequently mix it with the another effluent obtained from the second separator, as the other effluent still comprises spent, i.e. non-rejuvenated, ionic liquid catalyst.

The solids, which are removed from the process may be discarded, however it is preferred to reuse the components in the solids, for example in the preparation of fresh acidic ionic liquid catalyst.

In the process according to the invention, an isoparaffin and an olefin are reacted to form an alkylate by contacting the reaction mixture comprising isoparaffin and olefin with the catalyst under alkylation conditions.

Preferably, the hydrocarbon mixture comprises at least isobutane, isopentane or a mixture thereof as an isoparaffin. The hydrocarbon mixture preferably comprises at least an olefin comprising in the range of from 2 to 8 carbon atoms, more preferably of from 3 to 6 carbon atoms, even more preferably 4 or 5 carbon atoms. Examples of suitable olefins include, propene, 1-butene, 2-butene, isobutene, 1-pentene, 2-pentene, 2-methyl-1-butene, 3-methyl-1-butene, 2-methyl-2-butene.

Isoparaffins and olefins are supplied to the process in a molar ratio, which is preferably 1 or higher, and typically in the range of from 1:1 to 40:1, more preferably 1:1 to 20:1. In the case of continuous reaction, excess isoparaffin can be recycled for reuse in the hydrocarbon mixture.

The alkylation conditions (or process conditions) are those known in the art for HF and sulphuric acid alkylation. Actual operational process conditions are among others dependent of the exact composition of the hydrocarbon mixture and catalyst.

The temperature in the reactor is preferably in the range of from -20 to 100°C, more preferably in the range of from 0 to 50°C. The temperature must in any case be above the melting temperature of the ionic liquid.

To suppress vapour formation in the reactor, the process is performed under pressure, preferably the pressure in the reactor is in the range of from 0.1 to 1.6 MPa.

The hydrocarbon mixture may be contacted with the catalyst in any suitable alkylation reactor. The hydrocarbon mixture may be contacted with the catalyst in a semi-continues or continuous process.

### Detailed description of the drawings.

In Figure 1 a schematic representation is given of a process according to the invention.

In Figure 1, a hydrocarbon mixture, comprising olefin and isoparaffin, e.g. butene and isobutane, is provided to reactor 100 through line 105. Acidic ionic liquid catalyst is also provided to reactor 100 through line 110. In reactor 100, the hydrocarbon mixture and catalyst are mixed under alkylation conditions. Through line 115, an alkylate-comprising reactor effluent comprising solids is withdrawn from the reactor. Part of this effluent may be directly recycled to the reactor or combined with line 105 via recycle line 117. At least part of the effluent is supplied to first separation unit 120, e.g. a cyclone. In first separation unit 120, the reactor effluent is separated in an ionic liquid phase effluent and multiple-phase effluent under influence of the centrifugal or gravity forces. The ionic liquid phase effluent can be recycled to the reactor via line 130.

The multiple-phase effluent is provided via line 135 from first separation unit 120 to second separation unit 140, e.g. a cyclone. In second separation unit 140, the multiple-phase effluent is separated in to an hydrocarbon phase effluent and another effluent. The other effluent may withdrawn from second separation unit 140 via line 147, be combined with line 115 and recycled to the first separation unit. Part of the hydrocarbon phase effluent may be directly recycled to the reactor or combined with line 105 via recycle lines 117 and 119. At least part of the hydrocarbon phase effluent is provided to fractionator 125 through line 145. From the bottom of fractionator 125, an alkylate-comprising product is retrieved through line 127. The alkylate product can be used for instance for fuel blending purposes. Additionally, an stream comprising isoparaffin is retrieved from fractionator 125, which is recycled via line 129 to become part of the hydrocarbon mixture in line 105. Other hydrocarbon-comprising streams (not shown) may also be retrieved from fractionator 125.

Part or all of the ionic liquid phase effluent can be diverted from line 130 by line 150 to third separation unit 155, e.g. a centrifuge. In centrifuge 155, solids are removed from the ionic liquid phase effluent under influence of the centrifugal forces, and are retrieved via line 160. The remaining ionic liquid phase effluent exits centrifuge 155 via line 165. Optionally, hydrochloride gas is provided to the ionic liquid phase effluent via line 170 from gas container 175. Optionally, a mixing device (not shown), e.g. a venturi absorber, is used to mix the hydrogen chloride gas into line 165. By allowing part of the catalyst to bypass the hydrogen chloride rejuvenation via line 130, any remaining gaseous hydrogen chloride may react with the ionic liquid catalyst in line 130 when lines 165 and 130 come together. It is also possible to provide a bypass (not shown) around the intersection of lines 165 and 170. In this way solids may be removed also from the ionic liquid catalyst, which is not rejuvenated.

The ionic liquid phase effluent is subsequently directed back to reaction zone 100. If necessary additional fresh acidic ionic liquid catalyst or externally supplied spent acidic ionic liquid catalyst can be provided to reaction zone 100 via line 180.

### Examples

The invention is illustrated by the following nonlimiting examples.

### Example 1

In order to show the effectiveness of the process according to the invention wherein the hydrocarbon phase or obtained by at least two separation steps, a sample reactor effluent, comprising a mixture of hydrocarbon reactants, predominantly isobutane, and alkylate products and ionic liquid catalyst was separated using a cyclone.

The sample reactor effluent comprises hydrocarbons and ionic liquid catalyst in a volume ratio of 1:1.05. The ionic liquid was an ionic liquid catalyst comprising a coordinate anion derived from aluminium(III) chloride and copper(I) chloride) (ex China University of Petroleum Beijing).

The operating temperature was maintained between 30 and 50°C and the operating pressure was maintained between 0.1 to 0.5 MPa. The maximum feed rate of sample reactor effluent to the cyclone was 2 m³/hr.

### Example 1a (not according to the invention)

In this experiment, the sample reactor effluent was separated into a hydrocarbon phase effluent and an ionic liquid phase effluent using a single separation step. The obtained separation results are below.

In order to prevent a too high fraction of ionic liquid in the hydrocarbon phase effluent only 40vol% of the sample reactor effluent was retrieved as hydrocarbon phase effluent. In case of one separator, the fraction of the reactor effluent that is retrieved from the separator as the hydrocarbon phase effluent cannot be increased without increasing the fraction of ionic liquid comprised therein. This may lead to contamination of the alkylate product and increased ionic liquid losses.

The remaining 60 vol% of the sample reactor effluent was retrieved as ionic liquid phase effluent.

The hydrocarbon phase effluent comprised:
- 95.5vol% of hydrocarbons; and
- 4.5vol% of ionic liquid,
based on the volume of the hydrocarbon phase effluent.

The ionic liquid phase effluent comprised:
- 17.7vol% of hydrocarbons; and
- 82.3vol% of ionic liquid,
based on the volume of the ionic liquid phase effluent.

Using one separator approximately 79vol% of the hydrocarbons originally provided in the sample reactor effluent were recovered in the hydrocarbon effluent.

### Example 1b

In this experiment, the sample reactor effluent was separated into a hydrocarbon phase effluent and an ionic liquid phase effluent using two separation steps. The obtained separation results are below.

50 vol% of the feed. i.e. sample reactor effluent, to the centrifuge was retrieved as a, lower density, multiple phase effluent and the remaining 50 vol% as higher density ionic liquid phase effluent.

The multiple phase effluent comprised:
- 93.8vol% of hydrocarbons; and
- 6.2vol% of ionic liquid,
based on the volume of the multiple phase effluent.

The ionic liquid phase effluent comprised:
- 90.0vol% of hydrocarbons; and
- 10.0vol% of ionic liquid,
based on the volume of the ionic liquid phase effluent.

The obtained multiple phase effluent was retrieved as an intermediate product and subjected to a second cyclone separation step. During the second cyclone separation step, 85vol% of the multiple phase effluent was retrieved from the cyclone as, lighter density, hydrocarbon phase effluent and the remaining 15 vol% of the multiple phase effluent was retrieved as a higher density phase effluent, also referred to a (an)other effluent.

The hydrocarbon phase effluent comprised:
- 98.5vol% of hydrocarbons; and
- 1.5vol% of ionic liquid,
based on the volume of the hydrocarbon phase effluent.

The other effluent comprised:
- 68.7vol% of hydrocarbons; and
- 31.3vol% of ionic liquid,
based on the volume of the other effluent.

Using two separators approximately 86 vol% of the hydrocarbons originally provided in the sample reactor effluent were recovered in the hydrocarbon effluent. By using two separators, in this case cyclones, in series to separate the hydrocarbon phase effluent from the sample reactor effluent, an increased fraction of the hydrocarbons in the sample reactor effluent can be retrieved and sent to the fractionator. This is due to the fact that a larger fraction of the reactor effluent is obtained as the multiple phase effluent form the first separator. Although, passing a larger fraction of the sample reactor effluent to the lighter multiple phase effluent causes the ionic liquid fraction in this multiple phase effluent to increase, the final ionic liquid fraction in the resulting hydrocarbon phase effluent is much lower due to the second separator step. As a result a larger fraction of the hydrocarbons in the reactor effluent can be separated from the ionic liquid. In Example 1a, using only one separator, the hydrocarbon phase effluent comprises only 79 vol% of the hydrocarbons originally present in the reactor effluent. In Example 1b, however, 86 vol% of the hydrocarbons in the reactor effluent were retrieved in the hydrocarbon effluent, comprising less ionic liquid.

In addition, the ionic liquid phase effluent comprised less hydrocarbons compared to example 1a, even in the case it is combined with the (an)other effluent.

In case the hydrocarbon phase effluent is further treated in a further settler unit, the size of volume of the settler unit is only 42.5% of a settler unit used to separate the whole reactor effluent.

### Example 2:

This example shows the benefit of using at least one centrifugal separation unit, in this case a cyclone, instead of the conventional settler unit.

Based on a feed rate of 2 m3/hr to the cyclone, the residence time of the sample reactor effluent in the cyclone is 1.5 minutes. In order to obtain the same separation result using a conventional settler unit it would typically require a residence time of at least 15 minutes. As a consequence, in order to maintain the same feed rate, a conventional settler would be required having a volume of 0.5 m3, which is a volume increase by a factor 10 compared to the cyclone of example 1. As mentioned before this results in undesirable large inventories of liquefied light hydrocarbons.

## Claims

1. A process for preparing an alkylate comprising contacting in a reactor a hydrocarbon mixture comprising at least an isoparaffin and an olefin with an acidic ionic liquid catalyst under alkylation conditions to obtain an alkylate, which process further comprises:
- withdrawing an alkylate-comprising reactor effluent from the reactor, wherein the reactor effluent comprises an ionic liquid phase and a hydrocarbon phase;
- separating at least part the reactor effluent into an ionic liquid phase effluent and a multiple-phase effluent in a first separation unit;
- separating at least part of the multiple-phase effluent into a hydrocarbon phase effluent and another effluent in a second separation unit; and
- recycling at least part of the ionic liquid phase effluent to the reactor;
in which process the ionic liquid phase effluent comprises solids, which process further comprises:
- providing the ionic liquid phase effluent to a separation unit suitable for separating solids and liquids; and
- removing at least part of the solids from the ionic liquid phase effluent.

2. A process according to claim 1, wherein the first separation unit and/or the second separation unit is a settler separation unit or a centrifugal separation unit, preferably a centrifugal separation unit.

3. A process according to any one of the preceding claims, wherein the centrifugal separation unit is a cyclone separation unit.

4. A process according to any one of the preceding claims, wherein the ionic liquid phase effluent comprises ionic liquid phase and in the range of from 0 to 20 vol%, preferably of from 0 to 10 vol% of hydrocarbon phase based on the total liquid weight of the ionic liquid phase effluent.

5. A process according to any one of the preceding claims, wherein the hydrocarbon phase effluent comprises a hydrocarbon phase and in the range of from 0 to 3 vol% of ionic liquid phase, preferably 0 to 0.5 vol% of ionic liquid phase based on the total liquid weight of the hydrocarbon phase effluent.

6. A process according to any one of the preceding claims, further comprising:
- fractionating said hydrocarbon phase effluent into an at least a stream comprising alkylate and a stream comprising isoparaffin.

7. A process according to any one of the preceding claims, wherein the isoparaffin is isobutane and/or isopentane.

8. A process according to any one of the preceding claims, wherein the hydrocarbon mixture comprises an olefin comprising in the range of from 3 to 6 carbon atoms, preferably 4 or 5 carbon atoms.

9. Process according to any one of the preceding claims, wherein the acidic ionic liquid catalyst is a composite ionic liquid comprising of cations derived from a hydrohalide of an alkyl-containing amine or pyridine, anions being multiple-phase coordinate anions derived from two or more metal halides, wherein at least one metal halide is an aluminium halide and any further metal halide is a halide of a metal selected from the group consisting of Group IB elements of the Periodic Table, Group IIB elements of the Periodic Table and transition elements of the Periodic Table.

10. A process according to claim 9, wherein to the catalyst comprises aluminium chloride and copper (I) chloride or copper (II) chloride.

## Patentansprüche

1. Verfahren zur Herstellung eines Alkylats, umfassend das In-Kontakt-Bringen in einem Reaktor einer Kohlenwasserstoffmischung, umfassend mindestens ein Isoparaffin und ein Olefin mit einem Katalysator für saure ionische Flüssigkeiten unter Alkylierungsbedingungen, um ein Alkylat zu erhalten, wobei das Verfahren weiter Folgendes umfasst:
- Entnehmen eines Alkylat-haltigen Reaktorausflusses vom Reaktor, wobei der Reaktorausfluss eine ionische flüssige Phase und eine Kohlenwasserstoffphase umfasst;
- Trennen von mindestens einem Teil des Reaktorausflusses in einen Ausfluss der ionischen flüssigen Phase und einen Mehrphasen-Ausfluss in einer ersten Trenneinheit;
- Trennen von mindestens einem Teil des Mehrphasen-Ausflusses in einen Ausfluss der Kohlenwasserstoffphase und einen weiteren Ausfluss in einer zweiten Trenneinheit; und
- Recyceln von mindestens einem Teil des Ausflusses der ionischen flüssigen Phase an den Reaktor;
wobei in diesem Prozess der Ausfluss der ionischen flüssigen Phase Feststoffe enthält, wobei der Prozess weiter Folgendes umfasst:
- Bereitstellen des Ausflusses der ionischen flüssigen Phase an eine Trenneinheit, die geeignet ist, um Feststoffe und Flüssigkeiten zu trennen; und
- Entfernen von mindestens einem Teil der Feststoffe vom Ausfluss der ionischen flüssigen Phase.

2. Verfahren nach Anspruch 1, wobei die erste Trenneinheit und/oder die zweite Trenneinheit eine Settler-Trenneinheit oder eine zentrifugale Trenneinheit ist, vorzugsweise eine zentrifugale Trenneinheit.

3. Verfahren nach einem der vorhergehenden Ansprüche, wobei die zentrifugale Trenneinheit eine Zyklon-Trenneinheit ist.

4. Verfahren nach einem der vorhergehenden Ansprüche, wobei der Ausfluss der ionischen flüssigen Phase eine ionische flüssige Phase im Bereich von 0 bis 20 Vol%, vorzugsweise von 0 bis 10 Vol% der Kohlenwasserstoffphase umfasst, basierend auf dem gesamten Flüssigkeitsgewicht des Ausflusses der ionischen flüssigen Phase.

5. Verfahren nach einem der vorhergehenden Ansprüche, wobei der Ausfluss der Kohlenwasserstoffphase eine Kohlenwasserstoffphase im Bereich von 0 bis 3 Vol% der ionischen flüssigen Phase, vorzugsweise 0 bis 0,5 Vol% der ionischen flüssigen Phase umfasst, basierend auf dem gesamten Flüssigkeitsgewicht des Ausflusses der Kohlenwasserstoffphase.

6. Verfahren nach einem der vorhergehenden Ansprüche, weiter umfassend:
- Fraktionieren des Ausflusses der Kohlenwasserstoffphase in mindestens einen Strom, umfassend Alkylat, und einen Strom, umfassend Isoparaffin.

7. Verfahren nach einem der vorhergehenden Ansprüche, wobei das Isoparaffin Isobutan und/oder Isopentan ist.

8. Verfahren nach einem der vorhergehenden Ansprüche, wobei die Kohlenwasserstoffmischung ein Olefin umfasst, umfassend im Bereich von 3 bis 6 Kohlenstoffatome, vorzugsweise 4 bis 5 Kohlenstoffatome.

9. Verfahren nach einem der vorhergehenden Ansprüche, wobei der Katalysator für saure ionische Flüssigkeiten eine zusammengesetzte ionische Flüssigkeit ist, umfassend Kationen, abgeleitet von einem Hydrohalid eines Alkyl-haltigen Amins oder Pyridins, Anione, wobei es sich um mehrphasigen koordinierte Anionen handelt, abgeleitet von einem oder von mehreren Metallhaliden, wobei mindestens ein Metallhalid ein Aluminiumhalid ist und jedes weitere Metallhalid ein Halid eines Metalls ist, ausgewählt aus der Gruppe, bestehend aus Elementen der Gruppe IB des Periodensystems, Elementen der Gruppe IIB des Periodensystems und Übergangselementen des Periodensystems.

10. Verfahren nach Anspruch 9, wobei der Katalysator Aluminiumchlorid und Kupfer (I) chlorid oder Kupfer (II) chlorid umfasst.

## Revendications

1. Procédé de préparation d'un alkylat comprenant la mise en contact dans un réacteur d'un mélange hydrocarboné comprenant au moins une isoparaffine et une oléfine avec un catalyseur liquide ionique acide dans des conditions d'alkylation pour obtenir un alkylat, lequel procédé comprend en outre :
- l'extraction d'un effluent de réacteur comprenant l'alkylat du réacteur, l'effluent de réacteur comprenant une phase liquide ionique et une phase hydrocarbonée ;
- la séparation d'au moins une partie de l'effluent de réacteur en un effluent de phase liquide ionique et un effluent polyphasique dans une première unité de séparation ;
- la séparation d'au moins une partie de l'effluent polyphasique en un effluent de phase hydrocarbonée et un autre effluent dans une seconde unité de séparation ; et
- le recyclage d'au moins une partie de l'effluent de phase liquide ionique vers le réacteur ;
procédé dans lequel l'effluent de phase liquide ionique comprend des solides, lequel procédé comprend en outre :
- la fourniture de l'effluent de phase liquide ionique à une unité de séparation adéquate pour séparer les solides et les liquides ; et
- le retrait d'au moins une partie des solides de l'effluent de phase liquide ionique.

2. Procédé selon la revendication 1, dans lequel la première unité de séparation et/ou la seconde unité de séparation est une unité de séparation et de décantation ou une unité de séparation centrifuge, de préférence, une unité de séparation centrifuge.

3. Procédé selon l'une quelconque des revendications précédentes, dans lequel l'unité de séparation centrifuge est une unité de séparation cyclonique.

4. Procédé selon l'une quelconque des revendications précédentes, dans lequel l'effluent de phase liquide ionique comprend une phase liquide ionique et de 0 à 20 % en volume, de préférence, de 0 à 10 % en volume de phase hydrocarbonée sur la base du poids de liquide total de l'effluent de phase liquide ionique.

5. Procédé selon l'une quelconque des revendications précédentes, dans lequel l'effluent de phase hydrocarbonée comprend une phase hydrocarbonée et dans la plage de 0 à 3 % en volume de phase liquide ionique, de préférence, de 0 à 0,5 % en volume de phase liquide ionique sur la base de poids liquide total de l'effluent de phase hydrocarbonée.

6. Procédé selon l'une quelconque des revendications précédentes, comprenant en outre :
- le fractionnement dudit effluent de phase hydrocarbonée en au moins un courant comprenant l'alkylat et un courant comprenant l'isoparaffine.

7. Procédé selon l'une quelconque des revendications précédentes, dans lequel l'isoparaffine est de l'isobutane et/ou de l'isopentane.

8. Procédé selon l'une quelconque des revendications précédentes, dans lequel le mélange hydrocarboné comprend une oléfine comprenant de 3 à 6 atomes de carbone, de préférence, 4 ou 5 atomes de carbone.

9. Procédé selon l'une quelconque des revendications précédentes, dans lequel le catalyseur liquide ionique acide est un liquide ionique composite constitué de cations dérivés d'un hydrohalogénure d'une amine ou pyridine contenant un groupe alkyle, les anions étant des anions coordonnés polyphasiques dérivés de deux halogénures métalliques ou plus, dans lequel au moins un halogénure métallique est un halogénure d'aluminium et tout autre halogénure métallique est un halogénure d'un métal choisi dans le groupe constitué des éléments de groupe IB du Tableau Périodique, des éléments de groupe IIB du Tableau Périodique et des éléments de transition du Tableau Périodique.

10. Procédé selon la revendication 9, dans lequel le catalyseur comprend du chlorure d'aluminium et du chlorure de cuivre (I) ou du chlorure de cuivre (II).
